# EUROPEAN PATENT SPECIFICATION

(11) **EP 4 041 127 B1**
(45) Date of publication and mention of the grant of the patent: **26.11.2025**
(21) Application number: 20807927.7
(22) Date of filing: 09.10.2020
(51) Int. Cl.: A61M 5/142, A61F 2/02

(54) **CELL ENCAPSULATION DEVICES**
ZELLVERKAPSELUNGSVORRICHTUNGEN
DISPOSITIFS D'ENCAPSULATION DE CELLULES

(30) Priority: 10.10.2019 US 201962913549 P
(43) Date of publication of application: 17.08.2022
(73) Proprietor: W. L. Gore & Associates, Inc., Newark, DE 19711 (US)
(72) Inventor: SCOTTI, Christine, M., Newark, DE 19711 (US); CULLY, Edward, H., Newark, DE 19711 (US)
(74) Representative: HGF
(86) International application number: PCT/US2020/054980
(87) International publication number: WO 2021/072190

(56) References cited:
- EP-A1- 2 988 699
- EP-A1- 3 538 167
- WO-A1-2018/089401
- US-A1- 2003 060 871
- US-A1- 2008 208 325

## Description

### FIELD

The present invention relates generally to the field of medical devices and, in particular, to therapeutic devices for encapsulating and implanting cells into a patient.

### BACKGROUND

Biological therapies are increasingly viable methods for treating peripheral artery disease, aneurysm, heart disease, Alzheimer's and Parkinson's diseases, autism, blindness, diabetes and other pathologies. With respect to biological therapies in general, cells, viruses, viral vectors, bacteria, proteins, antibodies and other bioactive moieties may be introduced into a patient by surgical or interventional methods that place the bioactive moiety into a tissue bed of a patient. Conventionally, the bioactive moieties are first placed in a device that is then inserted into the patient. Alternatively, the device may be inserted into the patient first with the bioactive moiety added later.

Often, the device has a core (e.g., silicone), such that vascularization can only occur from an outer perimeter of the device. The distance between bioactive moieties introduced into these devices and an oxygen supply and nutrient source presents a problem for cell encapsulation because without the supply of adequate oxygen and nutrients within a relatively short period of time, the cells will begin to die. Thus, there is a need for devices that encapsulate cells and/or other biological moieties, while ensuring cell survival and growth.

EP3538167A1 describes an implantable containment apparatus for receiving and retaining a plurality of cells for insertion into a patient, such as into a tissue bed, is disclosed. The device includes a chamber having structural spacers therein to maintain an average distance between the first interior surface and the second interior surface of the chamber and to define at least one reservoir space for the placement of cells within the chamber.

WO2018/089401 describes an implantable containment apparatus for receiving and retaining a biological moiety or a therapeutic device within a tissue bed is disclosed. The device includes a shaping element to maintain the device in a generally toroidal configuration and to return the apparatus to that configuration after deformation. The apparatus can be placed in a host tissue with minimal trauma to the patient. Methods for implanting and using the apparatus are also disclosed.

### SUMMARY

The invention is defined according to appended claims 1 to 9.

Other examples described relate to a therapeutic device that includes a tubular member (e.g., tubular body) and a lumen extending therethrough. A wall of the therapeutic device includes a first permeable composite layer, a second permeable composite layer, and a reservoir between the first and second composite layers. The reservoir is configured to receive and contain a biological moiety.

The first composite layer includes a first cell permeable layer and a first cell impermeable layer and the second composite layer includes a second cell permeable layer and a second cell impermeable layer.

The therapeutic device has a first end and a second end. Each of the first end and the second end is open to the lumen so that vascularization occurs from the first cell permeable layer of the first composite layer and the second cell permeable layer of the second composite layer.

The reservoir has a thickness between 50 microns and 200 microns.

The biological moiety is a plurality of cells.

The plurality of cells is selected from prokaryotic cells, eukaryotic cells, mammalian cells, non-mammalian cells, stem cells and combinations thereof.

The device further includes a port in fluid communication with the reservoir.

Also described is an article including a plurality of therapeutic devices, each of which includes a tubular member (e.g., tubular body). The therapeutic devices are connected by connection members. Each therapeutic device contains a lumen extending therethrough. Each of the therapeutic devices has a first end and a second end. Each of the first end and the second end is open to the lumen. Each of therapeutic devices has a wall including: a first permeable composite layer, a second permeable composite layer, and a reservoir between the first and second composite layers. The reservoir is configured to receive and contain a biological moiety.

The therapeutic devices are interconnected by connection members at a first end or at a second end of the therapeutic devices.

The therapeutic devices are independently movable from each other.

The therapeutic devices are fluidly interconnected by connection members.

The reservoir has a thickness of 50 microns to 200 microns.

According to the appended claims, there is provided a device including a toroidal therapeutic device having a first opening therein and a hollow interior. The toroidal therapeutic device has a wall including: a first permeable composite layer, a second permeable composite layer, and a reservoir between the first and second composite layers. The reservoir is configured to receive and contain a biological moiety.

In some embodiments, the toroidal therapeutic device has a second opening on a side opposing the first opening and in substantial alignment therewith.

In some embodiments, the device further includes a second toroidal therapeutic device having a third opening therein positioned adjacent the second opening.

In some embodiments, the first and second toroidal therapeutic devices are fluidly interconnected by the second and third openings.

In some embodiments, the biological moiety is a plurality of cells.

In some embodiments, the plurality of cells is selected from prokaryotic cells, eukaryotic cells, mammalian cells, non-mammalian cells, stem cells and combinations thereof.

In some embodiments, the first and second composite layers each include a cell permeable layer and a cell impermeable layer.

In some embodiments, the device further includes a port in fluid communication with the reservoir.

### BRIEF DESCRIPTION OF THE DRAWINGS

The accompanying drawings are included to provide a further understanding of the disclosure and are incorporated in and constitute a part of this specification, illustrate embodiments, and together with the description explain the principles of the present disclosure.
FIG. 1 shows a cross-sectional view of a therapeutic device;
FIG. 2 shows a cross-sectional view of the first composite layer, the second composite layer and the reservoir of the therapeutic device of FIG. 1;
FIG. 3 shows an expanded view of a portion of the therapeutic device of FIG. 1;
FIG. 4 shows an end view of the lumen and layers of the therapeutic device of FIG. 1;
FIG. 5 shows a cross-sectional view of a therapeutic device;
FIG. 6 shows a cross-sectional view of a first composite layer, a second composite layer and a reservoir of the therapeutic device of FIG. 6;
FIG. 7 shows a perspective view of an article including a plurality of therapeutic devices connected by connection members;
FIG. 8 shows a perspective view of the article of FIG. 7 with fill and flush ports;
FIG. 9 shows a perspective view of a toroidal therapeutic device according to the appended claims;
FIG. 10 shows a perspective view of the vascularized therapeutic device of FIG. 9 according to the appended claims;
FIG. 11 shows a perspective view of two therapeutic devices of FIG. 9 stacked on top of each other according to the appended claims; and
FIG. 12 shows a cross-sectional view of a first composite layer, a second composite layer and a reservoir of the therapeutic device of FIG. 9 according to the appended claims.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting. The terms "therapeutic device" and "device" may be used interchangeably herein. The terms "permeable composite layer", "porous composite layer", and "composite layer" may be used interchangeably herein. Additionally, the terms "tubular member" and "tubular body" may be interchangeably within this disclosure.

Described herein are therapeutic devices for encapsulating biological moieties, where the therapeutic device(s) containing the biological moieties are implanted into a patient, such as into a tissue bed, to provide a biological therapy. Also described herein are methods for forming the therapeutic devices and for introducing the biological moieties into the devices. In some embodiments, the therapeutic device is tubular or substantially tubular in shape, which allows for vascularization from within the lumen as well as the from the exterior of the therapeutic device. In some embodiments, the therapeutic device includes a tubular member (e.g., tubular body) formed with composite layers, each composite layer having a cell impermeable layer and a cell permeable layer that enables vascularization and cellular ingrowth. In some embodiments, the composite layers are spaced apart from one another to define reservoir spaces for the retention of biological moieties.

In some embodiments, biological moieties suitable for encapsulation and implantation using the devices described herein include cells, viruses, viral vectors, gene therapies, bacteria, proteins, polysaccharides, antibodies and other bioactive moieties. For simplicity, herein the biological moiety is referred to as a cell, but nothing in this description limits the biological moieties to cells or to any particular type of cell, and the following description applies also to biological moieties that are not cells. In some embodiments, various types of prokaryotic cells, eukaryotic cells, mammalian cells, non-mammalian cells, and/or stem cells may be used with the cell encapsulation devices of the present invention. In some embodiments, the cells are microencapsulated within a biomaterial of natural or synthetic origin, including, but not limited to, a hydrogel material.

In some embodiments, the cells secrete a therapeutically useful substance. In some embodiments, such substances include hormones, growth factors, trophic factors, neurotransmitters, lymphokines, antibodies, or other cell products which provide a therapeutic benefit to the device recipient. Examples of such therapeutic cell products include, but are not limited to, insulin, growth factors, interleukins, parathyroid hormone, erythropoietin, transferrin, and Factor VIII. Non-limiting examples of suitable growth factors include vascular endothelial growth factor, platelet-derived growth factor, platelet-activating factor, transforming growth factors, bone morphogenetic protein, activin, inhibin, fibroblast growth factors, granulocyte-colony stimulating factor, granulocyte-macrophage colony stimulating factor, glial cell line-derived neurotrophic factor, growth differentiation factor-9, epidermal growth factor and combinations thereof. It is to be appreciated that throughout this disclosure, the terms "cell" or "cells" could be replaced by "biological moiety" or "biological moieties," respectively.

### I. Therapeutic Device with a Vascularized Lumen

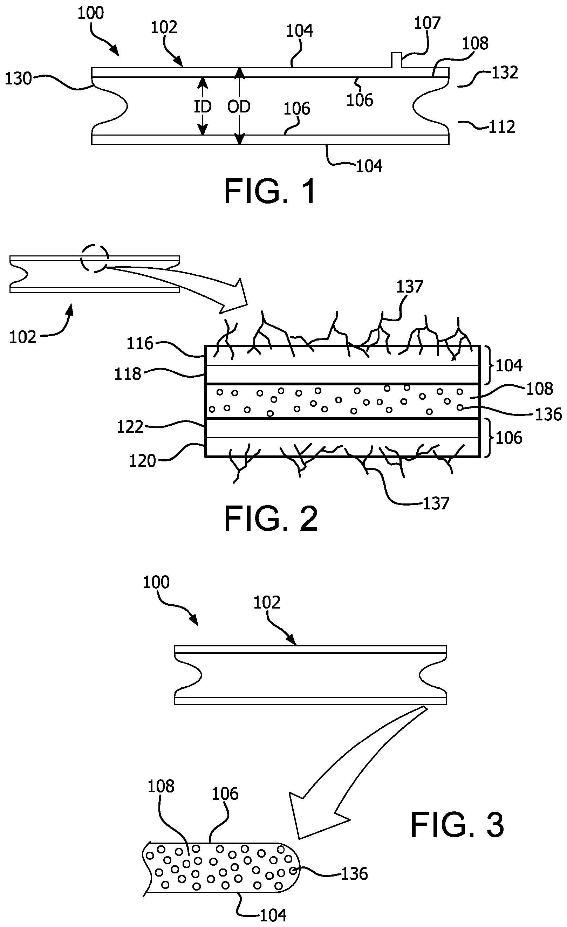

An example therapeutic device for encapsulating cells is illustrated in FIGS. 1-5. The therapeutic device 100 includes a tubular body 102 (e.g., tubular member) having an open first end 130 to an open second end 132. The body 102 is defined by a first permeable composite layer 104 and a second permeable composite layer 106 sealed along at least a portion of their periphery 110. A reservoir 108 for receiving cells is formed between the first and second permeable composite layers 104, 106. At least one port 107 is in fluid communication with the reservoir 108 and provides a path to access the reservoir 108 for filling, flushing or evacuating the reservoir 108. The port(s) 107 may be positioned anywhere on the body 102, so long as it provides a path to the reservoir 108 from the exterior of the therapeutic device 100. A lumen 112 extends through the tubular body 102, defining an inner diameter ID of the therapeutic device 100, while the outer surface of the first composite layer 104 defines an outer diameter OD of the therapeutic device 100, as shown in FIG. 1. The inner diameter may range from about 100 microns to about 5 mm, from about 150 microns to about 4.5 mm, from about 200 microns to about 4 mm, or from about 250 microns to about 3.5 mm. The lumen 112 extends through the entirety of the tubular body 102. The lumen 112 is substantially centrally located within the tubular body 102. The first composite layer 104, the second composite layer 106, and the reservoir 108 positioned therebetween define a wall of the tubular body 102. As will be described in further detail below, the lumen 112 of the therapeutic device 100 allows for ingrowth of vascular tissue from the inner diameter ID so that the distance between cells contained in the reservoir and the vascular tissue (*i.e*., nutrient source) is sufficient to maintain cell proliferation and growth.

As depicted in FIG. 2, the first composite layer 104 includes a cell permeable layer 116 and a cell impermeable layer 118 disposed adjacent to the cell permeable layer 116. The second composite layer 106 includes a cell permeable layer 120 and a cell impermeable layer 122. The cell permeable layers 116, 120 of the first and second composite layers 104, 106, respectively, may be formed of the same or different material.

The cell permeable layers 116, 120 have a pore size that is large enough to allow the ingrowth of a capillary network 137, as shown in FIG. 2. Herein, layers that have openings large enough to allow vascular ingrowth may be referred to as "cellular ingrowth" layers. The pore size of the cell permeable layers 116, 120 is greater than about 5.0 microns, as measured by porometry. Ingrowth of vascular tissues through both of the cell permeable layers 116, 120 (e.g., both through the cell permeable layer 120, which faces the lumen 112, and through the cell permeable layer 116, which faces the external environment surrounding the body 102) facilitates nutrient and biomolecule transfer through the cell permeable layer 116 from the patient up to but not across the cell impermeable layer 118.

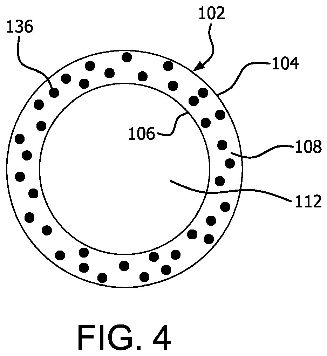

Various cell types can grow into the cellular ingrowth layers (*i.e*., cell permeable layers 116, 120) of the therapeutic device 100 as described herein. The predominant cell type that grows into a porous material depends primarily on the implantation site, the composition and permeability of the material, and any biological factors, such as, for example, cytokines and/or cell adhesion molecules that may be incorporated in the material or introduced through porous material(s). Vascular endothelium is the predominant cell type that grows into a porous material for use in a cell encapsulation device. Vascularization of the cell permeable layers 116, 120 by a well-established population of vascular endothelial cells in the form of a capillary network 137, as depicted in FIGS. 3-4, is encouraged to occur as a result of neovascularization from tissues of a patient into and across the thickness of the cell permeable layers 116, 120. FIG. 4 depicts an expanded view of the vascularization of a portion of the therapeutic device of FIG. 3.

The cell impermeable layers 118, 122, are impervious to vascularization and cell ingrowth and thus, are cell retentive layers. For example both cell impermeable layers 118, 122 have a pore size that is sufficiently small so as to prevent vascular ingrowth. However, the cell impermeable layers 118, 122 have a pore size that is sufficiently large to allow nutrients from the patient and products produced by the cells to pass therethrough. The pore size of the cell impermeable layers 118, 122 is less than about 5 microns, less than about 1 micron, or less than about 0.5 microns, as measured by porometry.

The outer cell permeable layers 116, 120 and/or the inner cell impermeable layers 118, 122 may be formed of alginate, cellulose acetate, polyalkylene glycols such as polyethylene glycol and polypropylene glycol, panvinyl polymers such as polyvinyl alcohol, chitosan, polyacrylates such as polyhydroxyethylmethacrylate, agarose, hydrolyzed polyacrylonitrile, polyacrylonitrile copolymers, polyvinyl acrylates such as polyethylene-co-acrylic acid, porous polytetrafluoroethylene (PTFE), porous modified polytetrafluoroethylene polymers, porous tetrafluoroethylene (TFE) copolymers, porous polyalkylenes such as porous polypropylene and porous polyethylene, porous polyvinylidene fluoride, porous polyester sulfone (PES), porous polyurethanes, porous polyesters, porous PPX (ePPX), porous ultra-high molecular weight polyethylene (eUHMWPE), porous ethylene tetrafluoroethylene (eETFE), porous vinylidene fluoride (eVDF), porous polylactic acid (ePLLA), and copolymers and combinations thereof, as well as woven or non-woven collections of fibers or yarns, or fibrous matrices, either alone or in combination. The cell permeable layers 116, 120 are porous expanded polytetrafluoroethylene membranes (e.g., ePTFE membranes).

The therapeutic device 100 includes only cell retentive layers (e.g., cell impermeable layers) and no cellular ingrowth layers (e.g., cell permeable layers), the therapeutic device 100 optionally could be used with a housing that is, or can be, implanted in a patient, and that is made from a vascularizing material that allows the ingrowth of vascular tissue. The housing may be implanted into a patient for a period of time sufficient to allow vascularization before the therapeutic device 100 is inserted into the housing. The therapeutic device 100 and the housing may be inserted into a patient together.

As noted above, the reservoir 108 is formed between the first composite layer 104 and the second composite layer 106 of the therapeutic device 100. As used herein, the term "reservoir" is meant to define the total area within the therapeutic device 100 between the cell impermeable layer 118 and the cell impermeable layer 122 and within the confines of the therapeutic device 100 where the placement of cells and other biological moieties occurs (and where the cells or other biological moieties reside). The reservoir 108 may include a plurality of reservoir subsections (not shown) for the placement of cells. The reservoir 108 includes two or more reservoir subsections that are interconnected so as to permit the flow of cells into and/or through the reservoir subsections. The reservoir 108 may take numerous configurations such as, but not limited to, a geometric shape (e.g., the general form of a rectangle, circle, square, semicircle, semi-oval, tube, etc.).

The "width" of the reservoir 108, as used herein, is meant to describe the distance between the cell impermeable layer 118 of the first composite layer 104 and the cell impermeable layer 122 of the second composite layer 106, as shown in FIG. 2 over a length of the therapeutic device 100 where the cells reside. The reservoir 108 has a thickness of at least 50 microns. The width is at least about 50 microns (e.g., between 50 microns and 100 microns), at least 100 microns (e.g. between 100 and 150 microns), or at least 150 microns (e.g., between 150 microns and 200 microns). It is to be noted that all ranges described herein are exemplary in nature and include any and all values in between..

The reservoir 108 is configured to hold cells 136 within the therapeutic device 100, as shown, for example, in FIG. 3. The therapeutic device 100 with the cells 136 therein is configured to be placed in a tissue bed of a patient to allow the cells 136 to provide biological therapy to the patient.

The cells 136 are introduced into the reservoir 108 of the therapeutic device 100 through one or more port(s) 107, such as is shown in FIG. 1. The port(s) 107 may be located in various regions along the body 102 of the therapeutic device 100 so long as the port(s) 107 extend through the first composite layer 104 to the reservoir 108 to permit cells 136 to be introduced into the reservoir 108. The cells 136 are introduced in the form of a suspension or slurry in a medium. The cells 136 may be individual cells, cell aggregates, or cell clusters. The medium may be a cell culture or cell growth medium, optionally including desired nutrients and/or other biomolecules. Insertion of the cells 136 through the port(s) 107 may be accomplished by a syringe.

The cells 136 may be introduced into the reservoir 108 prior to or after insertion of the therapeutic device 100 into a patient. For example, the therapeutic device 100 may be inserted into a patient and allowed to vascularize such that vascular tissue grows into the cell permeable layers 118, 122 of the device 100. The cells 136 may then be added while the therapeutic device 100 is *in vivo.* The cells 136 may be added to the reservoir 108 of the therapeutic device 100 prior to insertion of the therapeutic device 100 into a tissue bed of the patient.

As described above, the lumen 112 is encircled by the cell permeable layer 120, which promotes vascularization of the therapeutic device 100 from the lumen 112. Additionally, the cell permeable layer 116 promotes vascularization of the therapeutic device 100 from an exterior of the therapeutic device 100. Thus, vascularization occurs through both from within the lumen and from the exterior of the therapeutic device 100, as depicted in FIG. 2. The vascularization from both the lumen 112 of the therapeutic device 100 and the exterior of the therapeutic device 100 allows the volume of the cells contained in the reservoir 108 to increase because the cells are obtaining nutrients from both sides of the therapeutic device 100. The thickness of the reservoir 108 can expand in an amount from about 0.01 microns to about 400 microns. The thickness of the reservoir 108 can expand from about 0.01 microns to about 200 microns. The thickness of the reservoir can grow from about 0.01 microns to about 300 microns. The thickness of the reservoir can grow from about 0.01 microns to about 350 microns.

The therapeutic device 100 is made as described below. The following description is merely illustrative and thus, methods of forming the therapeutic device 100 is not limited to this description. The second composite layer 106 is formed by initially wrapping at least one layer (e.g., one layer, two layers, three layers, four layers, etc.) of a porous ePTFE membrane around a mandrel (e.g., a 4 mm stainless steel mandrel) to form the cell permeable layer 120 of the second composite layer 106. The porous membrane may be of the type described in U.S. Patent No. 5,814,405 to Branca, et al. A local heat source such as, for example, a soldering iron, is used to fix opposing edges of the porous ePTFE membrane to one another so that the cell permeable layer 120 takes the form of a tube. Next, at least one layer (e.g., one layer, two layers, three layers, four layers, etc.) of a cell retentive porous membrane are wrapped on the mandrel, over the cell permeable layer 120, to form the cell impermeable layer 122 of the second composite layer 106. The cell retentive porous membrane may be of the type described U.S. Patent No. 5,476,589 to Bacino. Subsequently, the local heat source is used to fix opposing edges of the cell retentive porous membrane to one another so that the cell impermeable layer 122 takes the form of a tube.

The mandrel is then heat treated (e.g., placed in an air convection oven), after which it is removed from the oven and allowed to cool to ambient temperature (e.g., approximately 20 °C). This heat treatment causes the membranes forming the second composite layer (i.e., the membrane(s) forming the cell permeable layer 120 and the membrane(s) forming the cell impermeable layer 122) to adhere to each other. The close contact with the mandrel minimizes distortion of the microstructure of the membranes.

Following formation of the second composite layer 106, the reservoir 108 is formed. To form the reservoir 108, spacers may be applied to the surface of the cell impermeable layer 122 of the second composite layer 106. The height of the spacers determines the depth of the reservoir 108. The depth of the reservoir 108 is selected depending on the cell type to be housed therein.

The first composite layer 104 is then similarly formed by wrapping at least one layer (e.g., one layer, two layers, three layers) of the cell retentive porous membrane around a larger mandrel (e.g., a 5 mm stainless steel mandrel), in a manner similar to the process described above, to form the cell impermeable layer 118. At least one layer (e.g., one layer, two layers, three layers, four layers, etc.) of the porous membrane are then wrapped on the same larger mandrel over the cell impermeable layer 118 to form the cell permeable layer 116 of the first composite layer 104. Respective opposing edges of the cell impermeable layer 118 and the cell permeable layer 116 are fixed to each other and the first composite layer 104 is heat treated as described above.

Once cool, the first composite layer 104 is removed from the mandrel and is placed over the second composite layer 106, which is still positioned on its mandrel, thereby forming a wall of the tubular body 102. At this point, the port(s) 107 may be arranged, if desired, so that they are in fluid communication with the reservoir 108. The heat source is then used to melt the ends of the first and second composite layers, causing the ends of the first and second composite layers 104, 106 to adhere and seal together. The first composite layer 104 is also adhered to the spacers positioned on the second composite layer 106 by the heat source, forming the tubular body of the therapeutic device. At this point, the therapeutic device 100 may be trimmed at each end.

The therapeutic device 100 is pressure tested to ensure that the seals at the ends of the therapeutic device 100 between the first and second composite layers 104, 106 are secure by applying pressure to the port(s) 107. When testing is complete, the therapeutic device 100 is stripped from the mandrel. The resultant therapeutic device 100 has a lumen for tissue ingress and ingrowth, inner and outer surfaces with open pores to allow for rapid vascularization, and a cell reservoir with tight pores to retain cells therein.

### II. Article Containing Multiple Therapeutic Devices Having Vascularized Lumens

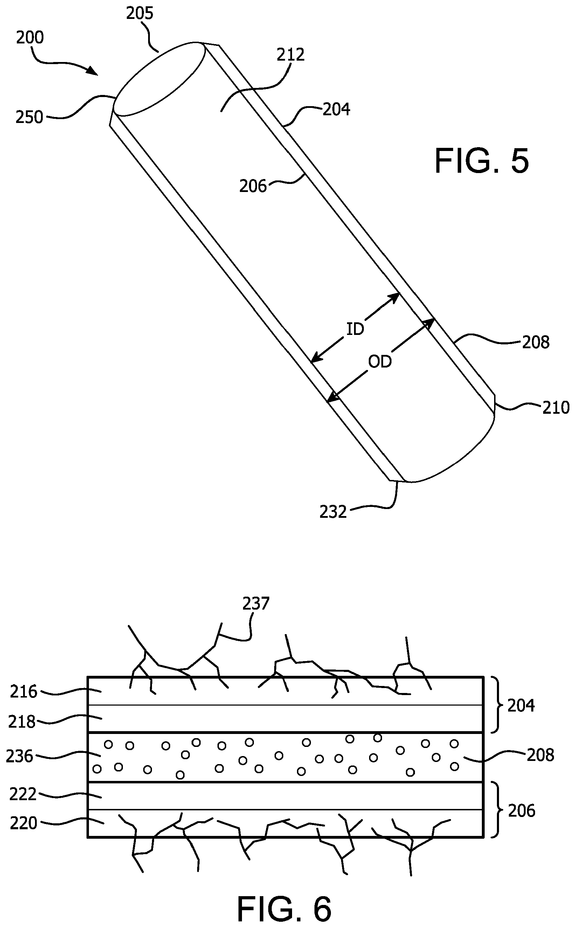

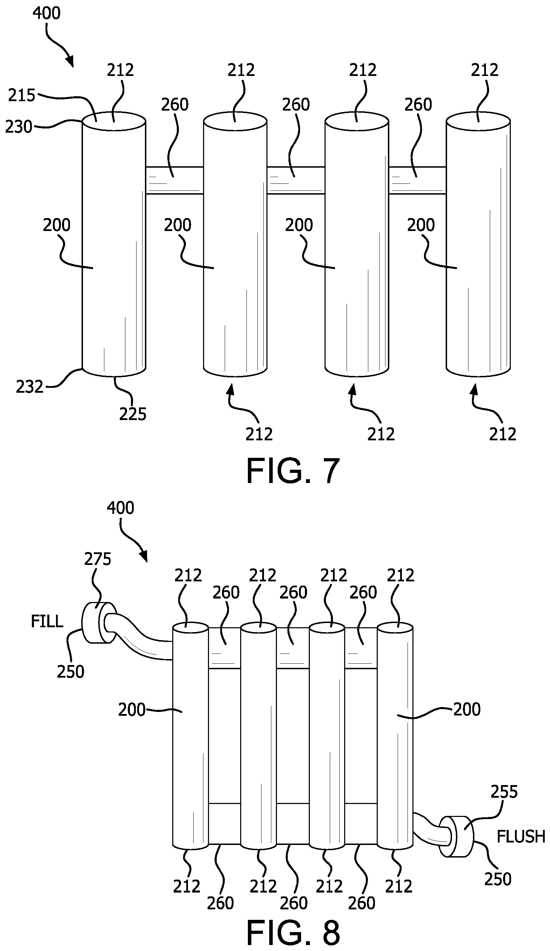

FIGS. 5-8 depict another example therapeutic device 200 and an article 400 that includes a plurality of therapeutic devices 200. As shown in FIG. 7, the plurality of therapeutic devices 200 are interconnected such that they are substantially parallel to each other along a length of the article 300. Turning to FIG. 5, each therapeutic device 200 includes a first open end 230 and a second open end 232. The therapeutic devices 200 include connection members 260 at their first ends 230. A lumen 212 extends through the tubular body 205 (e.g., tubular member) of the therapeutic device 202 from the first end 230 to the second end 232 such that the tubular body 205 is open at both ends 230, 232.

As shown in FIG. 5, the therapeutic device 200 includes a body 205, a first open end 230, and a second open end 232. A lumen 212 extends from the first open end 230 to the second open end 232. The therapeutic device 200 includes a first composite layer 204 and a second composite layer 206 sealed along at least a portion of their periphery 210. Referring now to FIG. 6, a reservoir 208 is formed between the first and second composite layers 204, 206. The first and second composite layers 204, 206 and the reservoir 208 positioned therebetween define a wall of the therapeutic device 200. A cross-section of the therapeutic device 200 may be, for example, circular, ovoid or elliptical. A lumen 212 extends through the entirety of the body 205 of the therapeutic device 200. An inner diameter ID of the therapeutic device 200 is defined by a cross-sectional diameter of the lumen 212. The outer surface of the first composite layer 204 defines an outer diameter OD of the therapeutic device 200 (see FIG. 5). As described above with respect to therapeutic device 100, the lumen 212 of the therapeutic device 200 allows for ingrowth of vascular tissue or a capillary network 237 into the cell permeable layer 220 of the second composite layer 206 from within the lumen 212. In addition, the cell permeable layer 216 of the first composite layer 204 promotes vascularization of the therapeutic device 200 from the exterior of the therapeutic device 200. Because a capillary network 237 is formed in the cell impermeable layers 216, 220, the distance between cells contained in the reservoir 208 and a nutrient source (i.e., vascular tissue or capillary network) is minimized, allowing cells within the reservoir 208 to grow at an increased rate.

As depicted in FIG. 6 the first composite layer 204 is a composite layer, substantially similar to first composite layer 104, and includes a cell permeable layer 216 and a cell impermeable layer 218 disposed adjacent to the cell permeable layer 216. Similarly, the second composite layer 206 is a composite layer, substantially similar to second composite layer 106, and includes a cell permeable layer 220 and a cell impermeable layer 222.

The cell permeable layers 216, 220 are substantially similar to cell permeable layers 116, 120 in that they are cellular ingrowth layers (e.g., cell permeable layers) and have at least the characteristics described above with respect to cell permeable layers 116, 120. The cell impermeable layers 218, 222 are substantially similar to cell impermeable layers 118, 122 in that they are cellular ingrowth layers (e.g., cell impermeable layers) and have at least the characteristics described above with respect to cell permeable layers 118, 122.

The cell permeable layers 216, 220 and the cell impermeable layers 218, 222 may include any of the materials listed above with regard to cell permeable layers 116, 120 and cell impermeable layer 118, 122. The reservoir 208 may be formed in substantially the same manner and have at least the characteristics described above with respect to reservoir 108. Specifically, the reservoir 208 is formed between the cell impermeable layers 218, 222 of the therapeutic device 200. The reservoir 208 is configured to hold cells 236 within therapeutic device(s) 200 of an article 400 that is placed in a tissue bed of a patient to allow the cells to provide a biological therapy to the patient. The cells 236 are introduced into the reservoir 208 of the therapeutic device 200 through first and second access ports 215, 225, which are depicted in FIG. 7.

The therapeutic devices 202 are scalable in that they can easily be configured (e.g., throughout a range of diameters, lengths, cross-sectional shapes, quantities of the therapeutic devices 200 within the article 400, etc.) so that the therapeutic devices 200 can be used to house cells with varying shapes and sizes while ensuring survival and function of these cells. The therapeutic devices 200 may be varied in diameter and length to achieve a desired amount of surface area required for optimum performance. The tubular configuration is designed to use as little area as possible within the host anatomy, yet provide adequate surface area for performance of the therapeutic devices 200 within the article 400.

Furthermore, the lumen 212 extending through the therapeutic device 200 decreases the distance between the cells in the reservoir 208 and their nutrient source, i.e., vascular tissue. Similar to the lumen 112 of the therapeutic device 100, the lumen 212 is encircled circumferentially by the cell permeable layer 220, which promotes vascularization of the therapeutic device 200 from the lumen 212. Additionally, the cell permeable layer 216 promotes vascularization of the therapeutic device 200 from an exterior of the therapeutic device 200. Thus, vascularization occurs through both from within the lumen 212 and from the exterior of the therapeutic device 200, as depicted in FIG. 6. This vascularization, or formation of a capillary network 237, from both the lumen 212 of the therapeutic device 200 and the exterior of the therapeutic device 200 allows the volume of the cells contained in the reservoir 208 toas much as double or more, because the cells are obtaining nutrients from the interior and the exterior of the therapeutic device 200.

The therapeutic devices 200 may have inner diameters that range from about 100 microns to about 5 mm, from about 150 microns to about 4.5 mm, from about 200 microns to about 4 mm, or from about 250 microns to about 3.5 mm. In some examples in which multiple therapeutic devices 200 are used, the therapeutic devices 200 are separated from each a distance from about 0.1 microns to about 3 mm, from about 5 microns to about 2.5 mm, from about 10 microns to about 2 mm, from about 25 microns to about 1.5 mm, or from about 50 microns to about 1 mm.

In some examples, such as the embodiment depicted in FIG. 7, the therapeutic devices 200 are independently movable with respect to each other, thus making the article 400 flexible and/or compliant with tissue and/or tissue movement. The reservoirs 208 of the therapeutic devices 200 are fluidly connected, such as by connection members 260 (depicted in FIGS. 7 and 8), so cells inserted into one therapeutic device 200 are able to flow into another, separate therapeutic device 200. The article 400 includes at least one filling port 275 in fluid communication with a therapeutic device 200 at a first end of the therapeutic device 200. The filling port 275 allows for the insertion of cells into the reservoirs 208 of the therapeutic devices 200. The article 400 may also include at least one flush port 255 in fluid communication with a therapeutic device 200 at a second end of a different therapeutic device 200, as depicted in FIG. 8. The filling port 275 and the flush port 255 may be positioned anywhere along the length of the therapeutic device 200, so long as they are in fluid communication with the reservoir 208. The therapeutic device includes a single port (not shown) which may be used for both filling and flushing. Once filled, the therapeutic devices 200 are sealed, as discussed below.

The filling port 275 and the flush port 255 can be repeatedly opened and closed with a seal 250. The seal 250 includes, but is not limited to, caps, plugs, clamps, compression rings or valves. The seal 250 may be attached to the filling port 275 or flush port 255 with friction, by clamping, or with a screw comprised of threads and grooves. Depending on the intended use of the article 400, the filling port 275 and/or the flush port 255 is sealed to create a hermetic seal or other fluid-tight seal. The article 400 is intended for permanent or long-term (e.g., at least three weeks) implantation in a patient.

### III. Therapeutic Device Having a "C"-Shaped Configuration

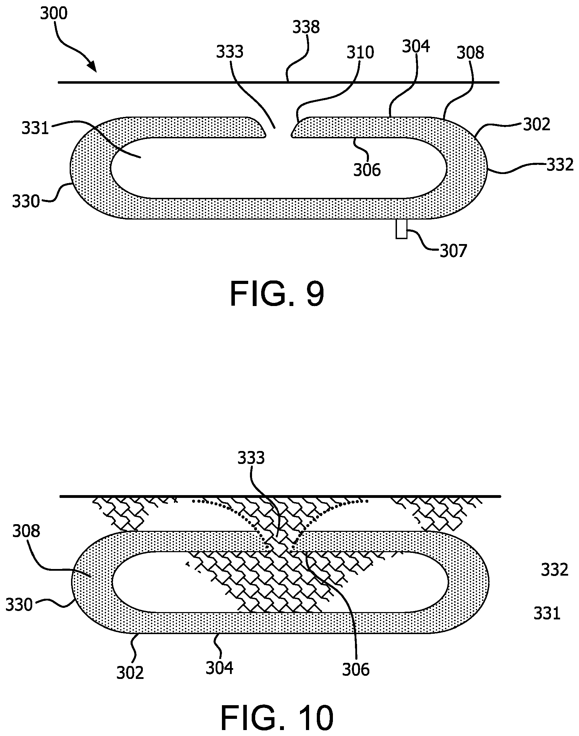

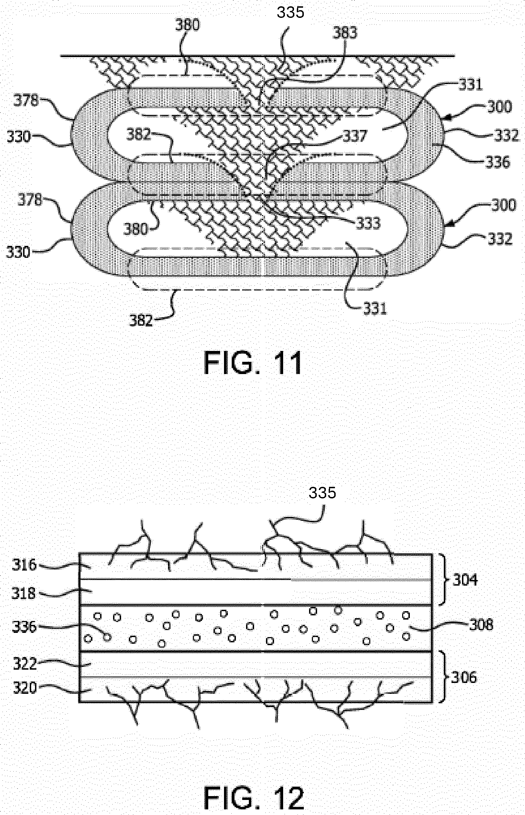

FIGS. 11-12 depict an embodiment of a therapeutic device 300 including a body 302 according to the appended claims. The therapeutic device 300 is substantially similar to therapeutic device 100 except that instead of having a tubular configuration, therapeutic device 300 is substantially C-shaped, as can be seen in FIGS. 9-10. Specifically, the body 302 may be substantially toroidal or ring-shaped about a central space 331, with an opening 333 extending through a side of the body 302. The body 302 is defined by a first composite layer 304 and a second composite layer 306 sealed along at least a portion of their periphery 310. A reservoir 308 is formed between the first and second composite layers 304, 306. The first and second composite layer 304, 306 and the reservoir 308 form a wall of the body 302. A port 307 is in fluid communication with the reservoir 308 and provides a path to access the reservoir 308 for filling, flushing or evacuating the reservoir 308. The port 307 may be positioned anywhere on the body 302, so long as it provides a path to the reservoir 308 from an exterior of the therapeutic device 300. The opening 333 allows for vascularization from the exterior of the body 302 of the therapeutic device 300 and from within the central space 331 (e.g., from both sides of the reservoir 308), decreasing the distance between cells contained in the reservoir 308 of the therapeutic device 300 and the nutrients provided by the vascular tissue, as will be described in further detail below.

As described above with respect to first and second composite layers 104, 106 and 204, 206, respectively, both the first composite layer 304 and the second composite layer 306 are sufficiently porous to permit the growth of vascular tissue from a patient into the pores of the cell permeable layer.

As depicted in FIG. 12, in some embodiments, the first composite layer 304, which is similar to the first composite layer 104 of the therapeutic device 100, includes a cell permeable layer 316 and a cell impermeable layer 318 disposed adjacent to the cell permeable layer 316. The second composite layer 306, which is similar to the second composite layer 106 of the first therapeutic device 100, includes a cell permeable layer 320 and a cell impermeable layer 322 positioned adjacent to the cell permeable layer.

The cell permeable layers 316, 320 are substantially similar to cell permeable layers 116, 120 in that they are cellular ingrowth layers and have at least the characteristics described above with respect to cell permeable layers 116, 120. The cell impermeable layers 318, 322 are substantially similar to the cell impermeable layers 118, 122 in that they are cellular ingrowth layers and have at least the characteristics described above with respect to cell impermeable layers 118, 122.

In some embodiments, the cell impermeable layers 318, 322 and the cell permeable layers 316, 320 contain the same material. For example, in some embodiments, the cell impermeable and cell permeable layers 318, 322 and 316, 320, respectively, include any of the materials listed above with respect to the cell impermeable and cell permeable layers 118, 122 and 116, 120, respectively.

The reservoir 308 is formed between the first composite layer 304 and the second composite layer 306 of the body 302 of the therapeutic device 300 in the same manner as the reservoir 108. The reservoir 308 is configured to hold cells 336 within a therapeutic device 300 placed in a tissue bed of a patient, as depicted in FIG. 12, to allow the cells 336 to provide a biological therapy to the patient. In some embodiments, the cells 336 are introduced into the reservoir 308 of the therapeutic device 300 through one or more port(s) 307. In some embodiments, the port(s) 307 extend through the first composite layer 304 of the body 302 of the therapeutic device 300 so that the cells 336 can be introduced into the reservoir 308.

As previously noted, the therapeutic device 300, in some embodiments, is substantially toroidal. The body 302 includes a central space 331 and an opening 333, allowing vascularization to occur both along the first composite layer 304 on the outer surface of the therapeutic device 300 and along the second composite layer 306 within the central space 331. Thus, the toroidal design of the therapeutic device 300 decreases the distance between the cells 336 in the reservoir 308 and their nutrient source, *i.e*., vascular tissue or a capillary network 335. Furthermore, vascularization from both the second composite layer 306 within the central space 331 of the therapeutic device 300 and the first composite layer 304 on the outer surface of the therapeutic device 300 allows the volume of the cells 336 contained in the reservoir 308 to, in some embodiments, increase by a factor of two or more, because the cells 336 are obtaining nutrients from both sides (i.e., inside and outside) of the therapeutic device 300.

In some embodiments, the therapeutic devices 300 are stackable, as shown in FIG. 11. As shown, each of the therapeutic devices 300 includes a first portion 380 of an outer surface 378 and an opposing second portion 382 of the outer surface 378. In these embodiments, at least two therapeutic devices 300 are positioned in a parallel configuration with a first portion 380 of the outer surface 378 of a first therapeutic device 300 abutting a second opposing portion 382 of the outer surface 378 of a second therapeutic device 300 such that the first and second ends 330, 332 of the therapeutic devices 300 are substantially aligned. In some embodiments, the first portion 380 of the outer surface includes the opening 333 while the second portion 382 does not include an open section. In other embodiments, each of the first portion 380 includes the opening 333 and the second portion 382 includes an opening 337. In such an embodiment, the openings 333, 337 are substantially aligned with one another such that vascularization can extend from the exterior of the therapeutic devices 300 through the central spaces 331 of the therapeutic devices 300, forming a linkage of vascularized therapeutic devices 300. In another embodiment, one therapeutic device 300 may have an opening 333 while a second therapeutic device 300 may have two openings 333, 337.

### IV. Bio-Absorbable Materials

In some embodiments, one or both composite layers of therapeutic devices 100, 200, 300 is or includes a bio-absorbable material. The bio-absorbable material may be formed as a solid (molded, extruded, or crystals), a self-cohered web, a raised webbing, or a screen. In some embodiments, one or more layers of bio-absorbable material are attached to a non-bio-absorbable material having macroscopic porosity to allow for cell permeation (e.g., a cell permeable layer) to form a composite. In other embodiments, a non-bio-absorbable material having microscopic porosity to decrease or prevent cell permeation is releasably attached to a porous self-cohered web to permit atraumatic removal of the therapeutic devices 100, 200, 300 from a patient in the days following implantation. Resorption into the patient can promote favorable type 1 collagen deposition, neovascularization, and a reduction of infection. In some embodiments, a screen is incorporated into a therapeutic device 100, 200, 300 to prevent "pillowing" of the device once the captive cells begin to multiply. In other examples, a bio-absorbable material could be incorporated into the cell encapsulation device as a powder. Non-limiting examples of suitable bio-absorbable materials include, but are not limited to, polyglycolide:trimethylene carbonate (PGA:TMC), polylactic acid, polyglycolic acid, poly (glycolide), poly(lactide-co-caprolactone), poly(caprolactone), poly(carbonates), poly(dioxanone), poly(hydroxybutyrates), poly(hydroxyvalerates), poly(hydroxybutyrates-co-valerates) and copolymers and blends thereof.

In some embodiments, incorporating bio-absorbable components into a therapeutic device 100, 200, 300 helps to facilitate implantation. For example, the bio-absorbable material may be, in some embodiments, temperature sensitive. In particular, the bio-absorbable material is much stiffer at colder temperatures and softens at higher temperatures (e.g., patient body temperature once implanted) so that the bio-absorbable material becomes more conformable and compliant after implantation. As a result, the longitudinal strength formed of a bio-absorbable material may allow a clinician to place the therapeutic device 100, 200, 300 in a patient with less effort and trauma to the host and, upon implantation, the bio-absorbable material becomes more conformable and compliant.

### V. Selective Permeability of Porous Layers

The porous material is porous only through a portion of its thickness such that the molecular weight cutoff, or sieving property, of the porous membrane begins at the surface. As a result, certain solutes and/or cells do not enter and pass through the porous spaces of the material from one side to the other. For example, the selective permeability of the porous material excludes cells from migrating or growing into the spaces of the porous material while permitting bi-directional flux of solutes across the thickness of the porous material. Vascular endothelial cells can then combine to form capillaries thereon. Such capillary formation or neovascularization of the porous material permits fluid and solute flux between tissues of a patient and the contents of the therapeutic device 100, 200, 300 to be enhanced.

Permeability of the porous material can be varied continuously across the thickness of the porous material of the therapeutic devices 100, 200, 300 described herein. For example, the selective permeability of the porous material can vary continuously across the thickness of the material. In some embodiments, the permeability of the porous material is varied from one cross-sectional area of the material to another to form a stratified structure.

The permeability of the porous material is varied across its thickness with additional layers of porous material. For example, in some therapeutic devices described herein, the selective permeability of the porous material is varied across the thickness of the porous material with one or more additional layers of porous material. Additional layers of porous material may have the same composition and permeability as the initial layer of porous material or the one or more additional layers may have a different composition and/or permeability.

The selective permeability of the porous material is varied by impregnating the void spaces of the porous material with a hydrogel material. A hydrogel material can be impregnated in all or substantially all of the void spaces of a porous material (*e.g*., pores of a porous membrane) or in only a portion of the void spaces. For example, by impregnating a porous material with a hydrogel material in a continuous band within the porous material adjacent to and/or along the interior surface of the porous material, the selective permeability of the porous material is varied from an outer cross-sectional area of the porous material to an inner cross-sectional area of the porous material.

The composition of hydrogel material impregnated into the porous material of the first and second composite layers depends in large part on the particular porous material used to construct an apparatus, the degree of permeability required for a given application, and the biocompatibility of the hydrogel material. Non-limiting examples of useful hydrogel materials for use in the therapeutic devices 100, 200, 300 include, but are not limited to, hydrolyzed polyacrylonitrile, alginate, agarose, carrageenan, collagen, gelatin, polyvinyl alcohol, poly(2-hydroxyethyl methacrylate), poly(N-vinyl-2-pyrrolidone), polyethylene glycol, polyethyleneimine, fibrin-thrombin gels, or gellan gum, and copolymers thereof, either alone or in combination. In certain aspects, the total thickness of a porous material (e.g. PTFE)/hydrogel composite may range from 30 µm to 1000 µm.

In yet other embodiments, the permeability of the porous material can be varied across the thickness of the porous material with an additional layer of porous material and a further layer of hydrogel material. One advantage of these embodiments is that this configuration will provide a strong cell and humoral immunoisolation barrier.

In some embodiments, the bio-absorbable material may have the capability to regenerate reactive oxygen species (ROS) at different levels in the patient's body. ROS have been shown to promote various cell responses in the patient's body including, but not limited to, inhibiting or promoting cell proliferation, differentiation, migration, apoptosis and angiogenesis.

In some embodiments, the materials used to construct the therapeutic devices 100, 200, 300, as described herein, are inherently radio-opaque. Those materials that are not inherently radio-opaque can be modified to be radio-opaque by impregnation of the materials with barium, for example. Other useful methods for rendering a material radio-opaque are known to those skilled in the art. The radio-opacity of materials used to construct a therapeutic device 100, 200, 300 as described herein is mainly used to facilitate surgical placement of the therapeutic device 100, 200, 300 or to locate the therapeutic device 100, 200, 300 in a patient following implantation.

### Example

A 50 mm long therapeutic device with a 4 mm inner diameter is described herein. The second composite layer is formed by initially wrapping three layers of an open porous ePTFE membrane around a 4 mm stainless steel mandrel to form the cell permeable layer of the second composite layer. The open porous membrane may be of the type described in U.S. Patent No. 5,814,405 to Branca et al. A local heat source such as, for example, a Weller Soldering Iron, is used to tack opposing edges of the open porous membrane to one another so that the cell permeable layer takes the form of a tube. In an embodiment, the membrane has an open pore structure with intermodal distances of approximately 10 to 30 microns. Next, two layers of a cell retentive porous membrane are wrapped on the steel mandrel, over the cell permeable layer, to form the inner porous layer of the second composite layer. The cell retentive porous membrane may be of the type described U.S. Patent No. 5,476,589 to Bacino. Subsequently, the local heat source is used to tack opposing edges of the cell retentive porous membrane to one another so that the inner porous layer takes the form of a tube. In this embodiment, the cell retentive membrane has a pore sizes in the range of 0.05 to 0.4 micrometers.

The mandrel is then placed in an air convection oven set at 365 °C for approximately 15 minutes, after which it is removed from the oven and allowed to cool to ambient temperature (e.g., 20 °C). This heat treatment causes the membranes forming the second composite layer to adhere together. The close contact with the mandrel minimizes the microstructure from distorting. To form the reservoir, spacers are then applied to the surface of the second composite layer. The spacers, in some embodiments, are adhesive dots of fluorinated ethylene propylene (FEP), arranged on the surface of the cell retentive layer. The height of the spacers will determine the depth of the reservoir, which will vary depending on the cell type to be house. The spacers are approximately 500 microns tall. A continuous band of FEP is also placed at each end of the inner porous layer for later adhesion of the first and second composite layers. In some embodiments, the bands of FEP are spaced approximately 50 mm apart.

The first composite layer is then similarly formed by wrapping two layers of the cell retentive porous membrane around a 5 mm stainless steel mandrel, as described above, to form the inner porous layer. Three layers of the open porous membrane are then wrapped on the same 5 mm mandrel over the inner porous layer to form the cell permeable layer of the first composite layer. Respective opposing edges of the inner porous layer and the cell permeable layer are tacked to each other, and the first composite layer is heat treated as described above.

After cooling to room temperature, the first composite layer is removed from the 5 mm mandrel and is pulled onto the second composite layer, which is still positioned on the 4 mm mandrel, thereby forming a wall of the tubular body. At this point, the port(s) are arranged so that they are in fluid communication with the reservoir space. The local heat source is then used to melt the FEP bands at the end of the second composite layer. The melted FEP will flow into the pores of the first and second composite layers and cause the ends of the first and second composite layer to adhere and seal together. The first composite layer is also adhered to the FEP spacers positioned on the second composite layer by the local heat source, forming the tubular body of the therapeutic device. At this point, the therapeutic device may be trimmed at each end.

The therapeutic device is pressure tested to ensure that the seals are viable by applying pressure to the port(s). When testing is complete, the therapeutic device is stripped from the mandrel. The resultant therapeutic device has a 4 mm lumen for tissue ingress and ingrowth, inner and outer surfaces with open pores to allow for rapid vascularization, and a cell reservoir with a tight pores to retain cells therein.

## Claims

1. A therapeutic device (300) comprising:
a first body (302) having a first toroidal shape, a first opening therein (333), and a first central space (331);
wherein the first body includes:
a first permeable composite layer (304);
a second permeable composite layer (306); and
a reservoir (308) positioned between the first and second permeable composite layers,
wherein the reservoir is configured to receive and contain therein a biological moiety, and
wherein the first opening enables vascularization from within the first central space and from an exterior of the first body.

2. The therapeutic device of claim 1, wherein the first body has a second opening (337) on a side opposing the first opening and in substantial alignment therewith.

3. The therapeutic device of claim 1 or claim 2, further comprising a second body (302) having a second toroidal shape and a third opening therein (337) positioned adjacent the second opening.

4. The therapeutic device of claim 3, wherein the first body and second body are fluidly interconnected by the second and third openings.

5. The therapeutic device of any one of claims 1-4, wherein the biological moiety is a plurality of cells.

6. The therapeutic device of claim 5, wherein the plurality of cells is selected from prokaryotic cells, eukaryotic cells, mammalian cells, non-mammalian cells, stem cells and combinations thereof.

7. The therapeutic device of any one of claims 1-6, wherein the first and second composite layers each include a cell permeable layer (316) and a cell impermeable layer (318).

8. The therapeutic device of any one of claims 1-7, comprising a port (307) in fluid communication with the reservoir.

9. The therapeutic device of claim 1, wherein the first body has a second opening opposing the first opening.

## Patentansprüche

1. Therapeutische Vorrichtung (300), umfassend:
einen ersten Körper (302), der eine erste Torusform, eine erste Öffnung darin (333) und einen ersten zentralen Raum (331) aufweist;
wobei der erste Körper Folgendes beinhaltet:
eine erste durchlässige Verbundschicht (304);
eine zweite durchlässige Verbundschicht (306); und
einen Behälter (308), der zwischen der ersten und der zweiten durchlässigen Verbundschicht positioniert ist,
wobei der Behälter dazu konfiguriert ist, darin eine biologische Einheit aufzunehmen und zu enthalten, und
wobei die erste Öffnung eine Vaskularisierung von innerhalb des ersten zentralen Raums und von einem Äußeren des ersten Körpers ermöglicht.

2. Therapeutische Vorrichtung nach Anspruch 1, wobei der erste Körper eine zweite Öffnung (337) auf einer Seite aufweist, die der ersten Öffnung gegenüberliegt und im Wesentlichen damit ausgerichtet ist.

3. Therapeutische Vorrichtung nach Anspruch 1 oder Anspruch 2, ferner umfassend einen zweiten Körper (302) mit einer zweiten Torusform und einer dritten Öffnung darin (337), die benachbart zu der zweiten Öffnung positioniert ist.

4. Therapeutische Vorrichtung nach Anspruch 3, wobei der erste Körper und der zweite Körper durch die zweite und die dritte Öffnung miteinander in Fluidverbindung stehen.

5. Therapeutische Vorrichtung nach einem der Ansprüche 1-4, wobei die biologische Einheit eine Vielzahl von Zellen ist.

6. Therapeutische Vorrichtung nach Anspruch 5, wobei die Vielzahl von Zellen ausgewählt ist aus prokaryotischen Zellen, eukaryotischen Zellen, Säugetierzellen, Nicht-Säugetierzellen, Stammzellen und Kombinationen davon.

7. Therapeutische Vorrichtung nach einem der Ansprüche 1-6, wobei die erste und die zweite Verbundschicht jeweils eine zelldurchlässige Schicht (316) und eine zellundurchlässige Schicht (318) beinhalten.

8. Therapeutische Vorrichtung nach einem der Ansprüche 1-7, umfassend einen Anschluss (307) in Fluidkommunikation mit dem Behälter.

9. Therapeutische Vorrichtung nach Anspruch 1, wobei der erste Körper eine zweite Öffnung aufweist, die der ersten Öffnung gegenüberliegt.

## Revendications

1. Dispositif thérapeutique (300) comprenant :
un premier corps (302) comportant une première forme toroïdale, une première ouverture dans celui-ci (333) et un premier espace central (331) ;
ledit premier corps comprenant :
une première couche composite perméable (304) ;
une seconde couche composite perméable (306) ; et
un réservoir (308) positionné entre les première et seconde couches composites perméables,
ledit réservoir étant configuré pour recevoir et contenir dans celui-ci
une fraction biologique, et
ladite première ouverture permettant une vascularisation à partir de l'intérieur du premier espace central et à partir de l'extérieur du premier corps.

2. Dispositif thérapeutique de la revendication 1, ledit premier corps comportant une deuxième ouverture (337) sur un côté opposé à la première ouverture et sensiblement aligné avec celle-ci.

3. Dispositif thérapeutique de la revendication 1 ou de la revendication 2, comprenant en outre un second corps (302) comportant une seconde forme toroïdale et une troisième ouverture dans celui-ci (337) positionnée de manière adjacente à la deuxième ouverture.

4. Dispositif thérapeutique de la revendication 3, ledit premier corps et ledit second corps étant interconnectés de manière fluidique par les deuxième et troisième ouvertures.

5. Dispositif thérapeutique de l'une quelconque des revendications 1-4, ladite fraction biologique étant une pluralité de cellules.

6. Dispositif thérapeutique de la revendication 5, ladite pluralité de cellules étant choisie parmi des cellules procaryotes, des cellules eucaryotes, des cellules mammifères, des cellules non mammifères, des cellules souches et des combinaisons de celles-ci.

7. Dispositif thérapeutique de l'une quelconque des revendications 1-6, lesdites première et seconde couches composites comprenant chacune une couche perméable aux cellules (316) et une couche imperméable aux cellules (318).

8. Dispositif thérapeutique de l'une quelconque des revendications 1-7, comprenant un orifice (307) en communication fluidique avec le réservoir.

9. Dispositif thérapeutique de la revendication 1, ledit premier corps comportant une deuxième ouverture opposée à la première ouverture.
